# EUROPÄISCHE PATENTSCHRIFT

(11) **EP 3 270 793 B1**
(45) Veröffentlichungstag und Bekanntmachung des Hinweises auf die Patenterteilung: **23.09.2020**
(21) Anmeldenummer: 17721644.7
(22) Anmeldetag: 03.05.2017
(51) Int. Cl.: A61B 17/02, A61B 90/50

(54) **AUTOMATISCH NACHSPANNBARER CHIRURGISCHER HALTEARM**
SURGICAL RETAINING ARM THAT CAN BE AUTOMATICALLY RETIGHTENED
BRAS DE MAINTIEN CHIRURGICAL AUTOMATIQUEMENT RESSERRABLE

(30) Priorität: 04.05.2016 DE 102016108371
(43) Veröffentlichungstag der Anmeldung: 24.01.2018
(73) Patentinhaber: Aesculap AG, 78532 Tuttlingen (DE)
(72) Erfinder: BECK, Thomas, 78591 Durchhausen (DE); SEYFRIED, Dominik, 78126 Königsfeld (DE); VOGTHERR, Robert, 78532 Tuttlingen (DE)
(74) Vertreter: Winter, Brandl, Fürniss, Hübner, Röss, Kaiser, Polte - Partnerschaft mbB
(86) Internationale Anmeldenummer: PCT/EP2017/060554
(87) Internationale Veröffentlichungsnummer: WO 2017/191197

(56) Entgegenhaltungen:
- WO-A2-2014/140316
- DE-A1-102010 035 407
- US-A1- 2009 247 819

## Beschreibung

Die Erfindung betrifft eine chirurgische Vorrichtung zum Stabilisieren von Gewebe oder zum Positionieren von Organen oder zum Positionieren und Halten von chirurgischen Instrumenten und Geräten während eines chirurgischen Eingriffs, mit einem Grundkörper und einem, insbesondere an dem Grundkörper befestigten oder befestigbaren, flexiblen Arm, insbesondere Gliederarm, welcher in unterschiedliche Positionen und/oder Lagen bringbar ist und welcher über einen Spannmechanismus in einer gewünschten Stellung arretierbar ist, wobei ein Spannen und/oder Lösen des Spannmechanismus selbsttätig und/oder mittels einer Energiequelle erfolgt. Außerdem betrifft die Erfindung einen Nachspannmechanismus für einen chirurgischen Arbeitsarm.

Bei vielen chirurgischen Eingriffen werden heutzutage kraftbetätigte, bewegliche Haltearme verwendet, welche bestimmte chirurgische Geräte oder Instrumente halten, bewegen oder steuern können. Beispielsweise können derartige bewegliche Haltearme mittels Druckluft aus einem arretierten Zustand in einen beweglichen Zustand gebracht werden und mittels Federkraft gespannt bzw. arretiert werden, wobei die Steuerung der Druckluft mittels einer elektrischen Schaltung und mittels elektrischen Betätigungselementen erfolgen kann. Ein derartiger kraftbetätigter, beweglicher Haltearm ist beispielsweise aus der DE 10 2013 102 628 A1 bekannt.

Der Haltearm der DE 10 2013 102 628 A1 besteht aus einer Vielzahl von einzeln beweglichen, halbkugelförmigen Gliedern und einem durch die Mitte dieser Glieder geführten Seil, dessen distales Ende an dem letzten beweglichen Glied des Arms befestigt ist und dessen proximales Ende durch einen Spannmechanismus in Längsrichtung bewegt werden kann. Die Glieder sind an dem proximalen Ende des Arms durch ein feststehendes Bauteil begrenzt, so dass die Glieder des Arms - wenn das Seil in Längsrichtung zurückgezogen wird - aneinander gepresst werden und der Arm dadurch arretiert wird. Wird das Seil hingegen in Richtung des distalen Endes bewegt, entsteht wiederum Spiel zwischen den Gliedern, so dass diese zueinander frei werden und der gesamte Arm bewegt werden kann.

Der Gliederarm der DE 10 2013 102 628 A1 ist wie viele andere gattungsgemäße Haltearme nur für den Einmal-Einsatz vorgesehen, da er aufgrund seiner Konstruktion und Geometrie nicht aufbereitet bzw. sterilisiert werden kann. Er besteht daher weitestgehend aus Kunststoff-Materialien und ist als Teil eines zweiteiligen Aufbaus an einem Grundkörper befestigt. Die einzelnen Glieder des beweglichen Arms unterliegen dabei bei der zur Arretierung des Arms notwendigen Zugkraftaufbringung einer gewissen Verformung und Stauchung, wodurch der gesamte Gliederarm im gespannten Zustand ein wenig kürzer wird. Das innenliegende Seil, welches die Kraft überträgt, dehnt sich wiederum und wird unter Belastung länger. Folglich gibt der Haltearm nach, wird locker und kann somit seinen arretierten Zustand nicht aufrechterhalten. Da die Stellmechanik der DE 10 2013 102 628 A1, welche durch Federkraft bewegt wird, auf einen mechanischen Anschlag fährt, können derartige Stauchungen der Gliederarmsegmente und Dehnungen des Seils nicht ausgeglichen werden.

Aus der Druckschrift DE 29 511 900 U1 ist ein doppelter Druckluftkolben zum Bewegen einer mittels Federkraft gespannten Mechanik bekannt, wobei es sich hierbei um keinen vergleichbaren Haltearm handelt, bei welchem viele Gliederarmsegmente mittels eines Zugseils gespannt werden, woraus die problematischen Längenänderungen im Betrieb resultieren. Somit ist auch in der DE 29 511 900 U1 kein Nachspannen zum Ausgleich von Dehnungen und Stauchungen vorgesehen.

Im Stand der Technik sind andererseits Haltearme bekannt, welche zum Spannen oder Nachspannen mittels einer manuellen Anzugsvorrichtung wie beispielsweise einer Schraubmechanik arbeiten. Diese Anzugsvorrichtungen stellen die Zugkraft und den damit verbundenen zur Erzeugung der Zugkraft benötigten Längenausgleich einmal zu Beginn der Anwendung ein. Derartige manuelle Anzugsvorrichtungen sind beispielsweise aus den Druckschriften US 6 464 629 B1, US 6 866 628 B2, US 7 736 307 B2 und US 6 506 149 B2 bekannt.

In diesen Druckschriften wird die Zugkraft zum Spannen des Zugseils in dem Arm und somit zum Arretieren des Gliederarms mittels einer manuell vom Anwender zu bedienenden Schraube oder eines anderen Betätigungselements aufgebracht, so dass die Zugkraft dann in dieser Einstellung zunächst einmal auf einen bestimmten Punkt fixiert ist. Auch bei derartigen manuellen Anzugsvorrichtungen schwindet aufgrund von elastischen und/oder plastischen Längenänderungen des Gliederarms bzw. aufgrund der Dehnung des Zugseils über einen gewissen Zeitraum hinweg jedoch die Spannkraft in einem gewissen Maße und der Gliederarm ist nicht mehr genauso fest arretiert wie zu Beginn. Es ist somit während der Anwendungsdauer eines solchen Gliederarmes nach einer gewissen Zeit bzw. nach bestimmten Zeitintervallen ein Betätigen der Schraube und somit ein manuelles Nachspannen des Arms erforderlich. Der Anwender hat in diesen Druckschriften dann die Möglichkeit, die Schraube nachzuziehen und den Arm manuell nachzuspannen.

Der Stand der Technik hat somit die Nachteile, dass ein Nachspannen des Gliederarms entweder nicht vorgesehen ist oder dieses in aufwändiger Weise manuell durch einen Anwender durchgeführt werden muss. Es existiert somit bei den bisher bekannten Lösungen kein Nachspannen des Gliederarms ohne Zutun des Anwenders.

Es ist somit Aufgabe der vorliegenden Erfindung, die Nachteile aus dem Stand der Technik zu vermeiden oder wenigstens zu mildern. Insbesondere soll eine anfangs eingestellte oder technisch festgelegte Spannkraft ohne Zutun des Anwenders aufrechterhalten werden und eine Arretierung eines chirurgischen Gliederarms somit über einen langen Zeitraum ohne Zutun des Anwenders gewährleistet werden.

Diese Aufgabe wird insbesondere durch eine chirurgische Vorrichtung nach Anspruch 1 und durch einen Nachspannmechanismus nach Anspruch 10 gelöst. Vorteilhafte Ausführungsformen und Weiterbildungen sind Teil der Unteransprüche.

Die Erfindung betrifft zunächst eine chirurgische Vorrichtung zum Stabilisieren von Gewebe oder zum Positionieren von Organen oder zum Positionieren und Halten von chirurgischen Instrumenten und Geräten während eines chirurgischen Eingriffs, mit einem Grundkörper und einem, insbesondere an dem Grundkörper befestigten oder befestigbaren, flexiblen Arm, insbesondere Gliederarm, welcher in unterschiedliche Positionen und/oder Lagen bringbar ist und welcher über einen Spannmechanismus in einer gewünschten Stellung arretierbar ist, wobei ein Spannen und/oder Lösen des Spannmechanismus selbsttätig und/oder mittels einer Energiequelle erfolgt und wobei in einem arretierten Zustand des flexiblen Arms der Spannmechanismus eine Nachspannreserve, insbesondere einen Nachspannreserveweg, aufweist und der flexible Arm zur Aufrechterhaltung seines arretierten Zustands, insbesondere durch eine Energieeinbringung von der Energiequelle oder selbsttätig, unter Ausnutzung der Nachspannreserve automatisch nachspannbar ist.

Erfindungsgemäß ist somit insbesondere eine zweiteilig aufgebaute chirurgische Vorrichtung bestehend aus einem Grundkörper und einem daran befestigten bzw. befestigbaren, flexiblen Gliederarm vorgesehen. Der Gliederarm ist dabei aus Kunststoff-Material gefertigt und für den Einmalgebrach bestimmt. Der Gliederarm ist grundsätzlich frei und in sich beweglich und kann bei Bedarf in einer beliebigen Position bzw. Lage über einen Spannmechanismus arretiert bzw. gespannt bzw. eingefroren werden. Das Spannen und/oder Lösen des Spannmechanismus kann selbsttätig, beispielsweise durch Federkraft oder mittels einer, insbesondere externen, Energiequelle erfolgen. Als externe Energiequellen sind dabei grundsätzlich hydraulische oder pneumatische Druckquellen oder eine elektrische Energiequelle denkbar.

Wenn das vorliegende System bzw. die vorliegende chirurgische Vorrichtung so ausgelegt ist, dass in einem Betriebspunkt bzw. in einem arretierten Zustand des flexiblen Arms eine Nachspannreserve/ ein Nachspannreserveweg/ eine Wegreserve vorliegt, und der flexible Arm zur Aufrechterhaltung seines arretierten Zustands unter Ausnutzung der Nachspannreserve automatisch nachspannbar ist, kann - ohne Zutun eines Anwenders - eine anfangs vorliegende Spannkraft aufrechterhalten werden und eine Arretierung des Gliederarms über einen langen Zeitraum gewährleistet werden. Dies hat den Vorteil, dass sich ein Anwender, insbesondere ein Arzt bzw. Operateur, grundsätzlich darauf verlassen kann, dass auch nach längerer Zeit eine geeignete Spannkraft des Spannmechanismus vorliegt und der arretierte Zustand ohne sein Zutun aufrechterhalten wird. Dies ist bei chirurgischen Eingriffen bzw. Operationen, in welchen die erfindungsgemäße chirurgische Vorrichtung zum Einsatz kommt, äußerst vorteilhaft.

Die erfindungsgemäße chirurgische Vorrichtung muss dabei so ausgelegt sein, dass die Nachspannreserve in allen möglichen Positionen bzw. Lagen des flexiblen Arms gegeben ist. Der Grundkörper und der flexible Arm müssen somit insoweit in ihrer Konstruktion und Auslegung aufeinander abgestimmt sein, dass der erfindungsgemäße Nachspannreserveweg in jeder Betriebsposition gegeben ist. Die automatische Nachspannung kann dabei selbsttätig, beispielsweise mittels Federkraft, oder durch Energie-/ bzw. Krafteinbringung von der Energiequelle erfolgen.

Dabei hat es sich insbesondere als vorteilhaft herausgestellt, wenn das Spannen des Spannmechanismus selbsttätig mittels Federkraft und das Lösen des Spannmechanismus mittels der Energiequelle erfolgt, wobei die Nachspannreserve durch eine in dem arretierten Zustand des flexiblen Arms vorliegende Federwegreserve ausgebildet ist.

Nach diesem Ausführungsbeispiel erfolgt somit das Spannen bzw. Arretieren des flexiblen Arms bzw. Gliederarms mittels Federkraft, während hingegen das Lösen des flexiblen Arms mittels der Energiequelle und insbesondere mittels einer Kraftaufbringung entgegen der Federspannkraft durchgeführt wird bzw. erfolgt. Der normale Zustand bzw. Grundzustand in diesem Ausführungsbeispiel ist somit der gespannte Zustand. Über ein Betätigungselement kann ein Anwender den gespannten bzw. arretierten Zustand durch Kraftaufbringung entgegen der Federkraft mittels der Energiequelle aufheben. Dieses Ausführungsbeispiel hat insbesondere den Vorteil, dass in dem arretierten Zustand keine Energie von der Energiequelle benötigt wird. Es liegt somit kein Energieverbrauch in dem arretierten Zustand vor.

Wenn nun die Feder zum Spannen des flexiblen Arms in dem arretierten Zustand bzw. anfänglichen Betriebspunkt des Arms noch eine Federwegreserve aufweist, wird jegliche durch Stauchung oder Dehnung hervorgerufene infinitesimale Längenänderung direkt bzw. unmittelbar bzw. simultan durch die Federkraft unter Ausnutzung der Federwegreserve automatisch ausgeglichen. Dies hat den Vorteil, dass das automatische Nachspannen in einfacher Weise mittels einer mechanischen Lösung vorgenommen wird. In anderen Worten ist das vorliegende System somit derart ausgelegt, dass in dem arretierten Zustand des flexiblen Arms Weg- und Spannungsreserven vorliegen und somit keine Federwegbegrenzung durch beispielsweise einen Anschlag gegeben ist. Somit kann der Spannmechanismus den flexiblen Arm auch bei fortschreitender Längenänderung durch Dehnungen oder Stauchungen über einen großen Zeitraum hinweg automatisch und mit der Längenänderung simultan nachspannen und die Arretierung aufrechterhalten. Es ist somit gemäß diesem Ausführungsbeispiel keine Positionsüberwachung bzw. Ansteuerung zum Aufrechterhalten des arretierten Zustands notwendig.

Ein anderes vorteilhaftes Ausführungsbeispiel ist gekennzeichnet durch ein elektrisches Steuergerät, welches in dem arretierten Zustand des flexiblen Arms eine Änderung, insbesondere eine Verminderung, in einem Spannzustand des Spannmechanismus erfasst und basierend auf dem Erfassungsergebnis, insbesondere durch eine Energieeinbringung von der Energiequelle, ein automatisches Nachspannen des Spannmechanismus steuert.

Erfindungsgemäß können somit sowohl das Spannen, als auch das Nachspannen, als auch das Lösen des Spannmechanismus durch eine Energieeinbringung/ Krafteinbringung bzw. durch eine Steuerung der Energieeinbringung/ Krafteinbringung von der Energiequelle durchgeführt werden. In anderen Worten erfolgen ein Spannen durch eine Krafteinbringung bzw. -aufbringung in Spannrichtung und ein Lösen durch eine Krafteinbringung bzw. -aufbringung entgegen der Spannrichtung bzw. in Gegenspannrichtung. Das Spannen und Lösen des Spannmechanismus wird jeweils auf Wunsch des Anwenders durchgeführt. Beispielsweise können entsprechende Betätigungselemente an der erfindungsgemäßen chirurgischen Vorrichtung vorgesehen sein.

In einem Spannzustand des Spannmechanismus erfasst eine Erfassungseinrichtung, beispielsweise ein Kraftsensor, des elektrischen Steuergeräts fortlaufend den gegenwärtigen bzw. aktuellen Spannzustand des Spannmechanismus. Basierend auf dem Erfassungsergebnis steuert das elektrische Steuergerät die Energie- bzw.

Krafteinbringung von der Energiequelle, so dass der Spannzustand dadurch über eine längere Zeit ohne Zutun des Anwenders aufrechterhalten werden kann.

In vorteilhafter Weise ist dabei ein elektrischer Stellmotor vorgesehen, welcher eine Erfassungseinrichtung, insbesondere einen Kraftsensor, aufweist, welche die Änderung, insbesondere die Verminderung, in dem Spannzustand des Spannmechanismus erfasst, und welcher auf der Grundlage einer Steuerung durch das elektrische Steuergerät und basierend auf dem Erfassungsergebnis der Erfassungseinrichtung den flexiblen Arm automatisch nachspannt.

Demnach ist in dem Grundkörper als eine Energiequelle ein elektrischer Stellmotor vorgesehen. Dieser Stellmotor wird von dem elektrischen Steuergerät angesteuert. Grundsätzlich kann der elektrische Stellmotor durch Kraftaufbringung in Spannrichtung den flexiblen Arm spannen bzw. arretieren und durch Kraftaufbringung entgegen der Spannrichtung den flexiblen Arm lösen bzw. beweglich machen. Dies kann wiederum von einem Anwender durch entsprechende Betätigungselemente eingestellt werden. In dem arretierten Zustand des Arms erfasst das elektrische Steuergerät, welches beispielsweise integriert in dem elektrischen Stellmotor vorgesehen sein kann, über beispielsweise einen Kraftsensor eine Änderung in dem Spannzustand des Spannmechanismus. Basierend auf dem Erfassungsergebnis wird die Energieeinbringung durch den elektrischen Stellmotor gesteuert oder geregelt.

In diesem bevorzugten Ausführungsbeispiel wird die Zugkraft bzw. Spannkraft somit mittels eines elektrischen (Stell-)Motors erzeugt, welcher mit einem Kraftsensor ausgerüstet ist. Bei einer durch Längenänderungen des Armes auftretenden Spannkraftveränderung wird mittels einer Steuerung die anfangs eingestellte Kraft wieder erzeugt. Der Stell-Motor erhält somit die erforderliche Spannkraft aufrecht.

Erfindungsgemäß kann die Nachspannreserve auch durch einen in dem Grundkörper, insbesondere mittels der Energiequelle, linear verschieblichen Anschlag ausgebildet sein bzw. freigegeben werden.

Eine derartige Ausbildung der Nachspannreserve kann dergestalt sein, dass ein Spannen des Spannmechanismus über eine Federkraft erfolgt und der Federweg in dem anfänglichen Betriebspunkt bzw. arretierten Zustand des Gliederarms zunächst durch einen Anschlag begrenzt ist. Während des Betriebs der erfindungsgemäßen chirurgischen Vorrichtung, insbesondere während des gespannten Zustands, wird der Spannzustand über die Erfassungseinrichtung des elektrischen Steuergeräts überwacht. Liegt gemäß einem Erfassungsergebnis keine ausreichende Spannung mehr vor, kann der Anschlag mittels der Energiequelle linear in Spannrichtung verschoben werden. Ein Nachspannen erfolgt dann einhergehend mit der Verschiebung des Anschlags über die vorliegende bzw. dadurch freigegebene Federwegreserve.

Andererseits kann eine derartige Ausbildung der Nachspannreserve auch dergestalt sein, dass ein Spannen des Spannmechanismus durch Energieeinbringung pneumatisch, hydraulisch oder elektromotorisch vorgenommen wird und ein Verfahrweg einer beispielsweise verwendeten Stellmechanik durch den Anschlag begrenzt wird. Wird nun erfasst, dass ein Nachspannen erforderlich ist, kann der Anschlag linear verschoben werden und somit durch eine weitere Energieeinbringung der gewünschte bzw. geeignete Spannungszustand wiederhergestellt werden. Auch eine Zeitsteuerung bzw. ein zeitabhängiges Nachspannen ist erfindungsgemäß denkbar.

In vorteilhafter Weise weist der Spannmechanismus ein Zugseil, welches durch eine Vielzahl von zueinander beweglichen Gliederelementen des flexiblen Arms geführt ist und über welches die Gliederelemente reibschlüssig gegeneinander verspannbar sind, und eine selbsttätig und/oder mittels der Energiequelle betätigbare Stellmechanik zum Betätigen des Zugseils auf.

Erfindungsgemäß ist der flexible Arm der chirurgischen Vorrichtung somit derart ausgebildet, dass an einem distalen Ende ein Halteelement vorgesehen ist, an dem sich zu dem proximalen Ende des flexiblen Arms hin eine Vielzahl an halbkugelförmigen Gliederelementen anschließen, durch deren Mitte ein Seil geführt ist. Das Seil kann distal beispielsweise an dem Halteelement oder dem letzten beweglichen Gliederelement des Arms befestigt sein. An dem proximalen Ende des Arms sind zum einen die Gliederelemente durch ein feststehendes Bauteil begrenzt, zum anderen ist das Seil an einer Stellmechanik befestigt, welche in dem Grundkörper verschieblich angeordnet ist. Wird nun die Stellmechanik zusammen mit dem proximalen Ende des Seils in proximale Richtung hin bewegt, werden die Vielzahl an Gliederelementen zwischen dem Halteelement bzw. dem letzten beweglichen, distalen Gliederelement des Arms und dem feststehenden Bauteil verspannt bzw. eingeklemmt. Der Arm befindet sich dann in seinem arretierten, unbeweglichen Zustand.

In diesem Zustand muss erfindungsgemäß eine Nachspannreserve bzw. ein Nachspannreserveweg vorliegen. Die Stellmechanik darf somit in dem anfänglichen gespannten bzw. arretierten Zustand bzw. in dem Betriebspunkt nicht bereits gegen einen Anschlag gefahren sein. Hieraus wird deutlich, dass die einzelnen Bestandteile der erfindungsgemäßen chirurgischen Vorrichtung präzise aufeinander abgestimmt sein müssen. Insbesondere muss in allen möglichen Lagen bzw. Positionen des flexiblen Arms ein Mindestnachspannreserveweg vorgesehen sein. Erfindungsgemäß wird dabei angestrebt, Längenänderungen, welche durch Stauchungen der Gliederelemente und durch eine Dehnung des Seils hervorgerufen werden, von bis zu 5%, vorzugsweise bis zu 10%, in jedem Fall ausgleichen zu können. Es ist somit ein zentraler Aspekt der vorliegenden Erfindung, das Seil, die einzelnen Gliederelemente, die Spannkraft bzw. den Spannmechanismus und den Grundkörper unter anderem derart in konstruktiver Hinsicht geeignet auszulegen, dass dieser Mindestnachspannreserveweg in jedem Fall gewährleistet werden kann.

Es ist somit zweckmäßig, wenn die Stellmechanik innerhalb des Grundkörpers zum Spannen und/oder Lösen des Spannmechanismus linear beweglich ist und die Nachspannreserve durch eine in dem arretierten Zustand des flexiblen Arms weiterhin vorliegende lineare Verschieblichkeit der Stellmechanik in Spannrichtung auf einem Nachspannreserveweg ausgebildet ist. Außerdem ist es zweckmäßig, wenn die Stellmechanik hydraulisch, pneumatisch oder elektromotorisch, insbesondere mittels der von dem elektrischen Steuergerät angesteuerten Energiequelle betätigbar ist und das elektrische Steuergerät somit einen Fluiddruck und/oder eine wirkende elektrische Leistung an der Stellmechanik steuert oder regelt.

Ein vorteilhaftes Ausführungsbeispiel ist dadurch gekennzeichnet, dass die Stellmechanik nach Art einer Zylinder-Kolben-Mechanik ausgebildet ist, mit zumindest einem, vorzugsweise zwei Kolbenräumen, in welchen eine hydraulische oder pneumatische Energie zum Spannen und/oder Lösen und/oder Nachspannen des Spannmechanismus einbringbar ist, wobei das Spannen und Nachspannen des Spannmechanismus vorzugsweise ausschließlich über zumindest eine, vorzugsweise zwei Druckfedern oder Zugfedern erfolgt.

In einer bevorzugten erfindungsgemäßen Ausführungsform ist die Stellmechanik nach Art einer Zylinder-Kolben-Mechanik ausgebildet und weist einen, zwei oder mehr Kolbenräume bzw. Betätigungskammern auf, welche in Längsrichtung hintereinander in einem in dem Grundkörper vorgesehenen Zylinder angeordnet sind. Die Kolbenräume, in welchen jeweils ein Kolben verschiebbar gelagert ist, sind mit einem pneumatischen oder hydraulischen Medium befüllbar. Die vorzugsweise zwei Kolben sind mit einem gemeinsamen Betätigungsglied verbunden, wobei das Betätigungsglied wiederum mit dem Zugseil verbunden ist. Die Kolben beaufschlagen das Betätigungsglied vorzugsweise bei einer gleichzeitigen Befüllung der Kolbenkammern in gleicher Richtung mit einer Kraft. Durch das Verwenden von insbesondere zwei Kolben wird dabei eine größere wirksame Kolbenfläche erreicht, so dass eine Verstärkung der auf das Betätigungsglied wirkenden Kräfte zustande kommt.

In dieser Ausführungsform sind zum Spannen bzw. Arretieren des flexiblen Arms zwei Druckfedern vorgesehen. Es sind jedoch auch Zugfedern denkbar. Das Spannen sowie auch das Nachspannen des flexiblen Arms erfolgt dabei vorzugsweise ausschließlich über die Federn. Das Vorsehen von zwei Federn ermöglicht eine erhöhte Sicherheit, beispielsweise in dem theoretisch möglichen Fall des mechanischen Versagens von einer Feder. Das Lösen des Arms erfolgt auf der anderen Seite vorzugsweise ausschließlich über die pneumatische oder hydraulische Betätigung. Durch das Verwenden eines Doppelkolbens wird dabei ermöglicht, dass eine geringere Kraft bzw. Energie zum Lösen des Arms im Vergleich zu lediglich einem Kolben aufzubringen ist. Es ist somit eine Energieeinsparung möglich. Dadurch, dass das Spannen sowie Nachspannen ausschließlich in mechanisch einfacher Weise durch Federkraft erfolgt, ist keine aufwändige Steuerung der Spannkraft vonnöten. Die Federwegreserve, welche in dem anfänglichen Betriebspunkt vorgesehen ist, wird in anderen Worten bei jeder infinitesimal kleinen Längenänderung durch Dehnung des Seils oder Stauchung der Gliederelemente, unmittelbar von den beiden Druckfedern ausgenutzt und es erfolgt in mechanisch einfacher Weise eine mit der Längenänderung simultane, automatische Nachspannung.

In dieser bevorzugten Ausführungsform wird somit eine Zugkraft zum Spannen des Haltearms mittels Federkraft erzeugt, indem eine mit dem Zugseil verbundene Kolbenmechanik durch eine Druckfeder in entgegengesetzte Längsrichtung zum Gliederarm bewegt wird. Wenn dieses System so ausgelegt ist, dass die durch die Druckfeder bewegte Kolbenmechanik nach einem anfänglichen Längenausgleich nicht auf einen mechanischen Anschlag fährt, sondern darüber hinaus noch Weg- bzw. Spannungsreserven besitzt, kann der erfindungsgemäße Nachspannmechanismus den Gliederarm auch bei weiterer Längenänderung über einen großen Zeitraum hinweg automatisch nachspannen, so dass die Arretierung des Arms aufrechterhalten wird. Die Kolbenmechanik kann erfindungsgemäß aus einem, zwei oder mehr Kolben, aus einem, zwei oder mehr Kolbenräumen und aus einer, zwei oder mehr Druckfedern oder Zugfedern bestehen. Ein Lösen bzw. Freigeben des Gliederarms erfolgt hydraulisch oder pneumatisch.

Andererseits ist es erfindungsgemäß auch denkbar, dass das Spannen und Nachspannen des Spannmechanismus zusätzlich zu einer Federkraft durch Einbringen der hydraulischen oder pneumatischen Energie, insbesondere durch eine Kraftaufbringung in Spannrichtung, erfolgt und das Lösen des Spannmechanismus ausschließlich durch Einbringen der hydraulischen oder pneumatischen Energie, insbesondere durch eine Kraftaufbringung entgegen der Spannrichtung, erfolgt. Die Federkraft kann somit beim Spannen und Nachspannen auch durch eine hydraulische oder pneumatische Energie unterstützt werden, was wiederum eine erhöhte Sicherheit zur Folge hat.

Außerdem betrifft die Erfindung einen Nachspannmechanismus für einen chirurgischen Arbeitsarm, welcher einen Grundkörper und einen flexiblen Arm, insbesondere Gliederarm, aufweist, wobei der flexible Arm in unterschiedliche Positionen und/oder Lagen bringbar ist, wobei der Nachspannmechanismus insbesondere zum Einsatz in einer chirurgischen Vorrichtung wie voranstehend beschrieben ausgebildet ist und wobei der Nachspannmechanismus den in einem arretierten Zustand befindlichen flexiblen Arm zur Aufrechterhaltung seines arretierten Zustand automatisch nachspannt.

Insbesondere sieht der Nachspannmechanismus in einem arretierten Zustand des flexiblen Arms eine Nachspannreserve, insbesondere einen Nachspannreserveweg, vor und spannt den flexiblen Arm zur Aufrechterhaltung seines arretierten Zustands, insbesondere durch eine Energieeinbringung von einer Energiequelle oder selbsttätig, unter Ausnutzung der Nachspannreserve automatisch nach.

Mit anderen Worten betrifft die Erfindung ein automatisches Nachspannen eines chirurgischen Haltearms/ Gliederarms. Insbesondere soll erfindungsgemäß eine anfangs eingestellte oder technisch festgelegte Zugkraft bzw. Spannkraft aufrechterhalten werden und ein chirurgischer Gliederarm über einen langen Zeitraum ohne Zutun eines Anwenders arretiert werden. Anders ausgedrückt beinhaltet die Erfindung vorzugsweise eine Zugkraft-Mechanik mit Spann-Reserve im Betriebspunkt, um durch Dehnungen und Stauchungen bewirkte Längenänderungen auszugleichen und die Zugkraft bzw. Spannkraft dauerhaft und automatisch aufrecht zu erhalten.

Die Erfindung wird nachfolgend mit Hilfe von Figuren weiter erläutert. Es zeigen:
- Fig. 1: eine Längsschnittansicht der erfindungsgemäßen chirurgischen Vorrichtung gemäß einer ersten bevorzugten Ausführungsform in einem beweglichen Zustand des Gliederarms;
- Fig. 2: eine Längsschnittansicht der erfindungsgemäßen chirurgischen Vorrichtung gemäß der ersten Ausführungsform von Fig. 1 in einem arretierten Zustand des Gliederarms;
- Fig. 3: eine vergrößerte Ansicht des Spannmechanismus der erfindungsgemäßen chirurgischen Vorrichtung gemäß der ersten Ausführungsform von Fig. 1 und Fig. 2;
- Fig. 4: eine Längsschnittansicht der erfindungsgemäßen chirurgischen Vorrichtung gemäß einer zweiten bevorzugten Ausführungsform; und
- Fig. 5: eine Längsschnittansicht der erfindungsgemäßen chirurgischen Vorrichtung gemäß einer dritten bevorzugten Ausführungsform in einem beweglichen Zustand des Gliederarms.

Die Figuren sind lediglich schematischer Natur und dienen ausschließlich dem Verständnis der Erfindung. Gleiche Elemente sind dabei mit denselben Bezugszeichen versehen.

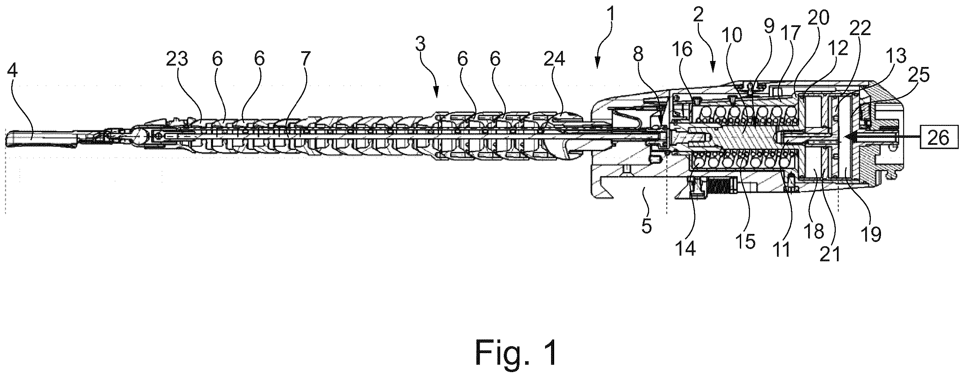

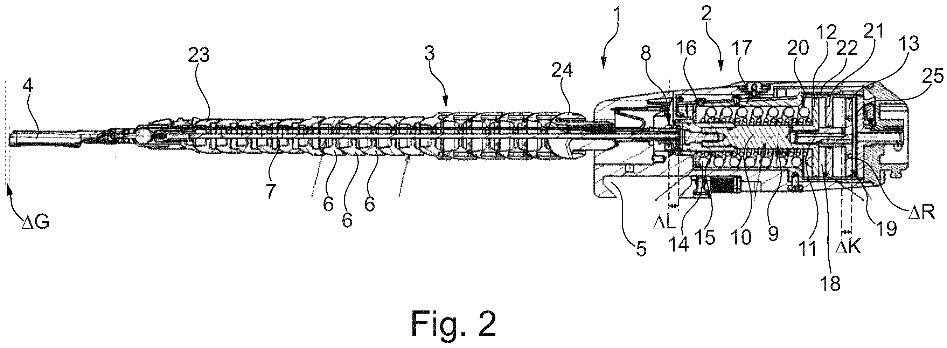

In Fig. 1 und Fig. 2 ist eine chirurgische Vorrichtung 1 nach einer bevorzugten Ausführungsform der vorliegenden Erfindung dargestellt. Die Vorrichtung 1 weist einen Grundkörper bzw. Technikblock 2 auf, an dem ein flexibler Gliederarm 3 befestigt ist. An dem freien, distalen Ende des Gliederarms 3 ist ein Halteelement 4 vorgesehen. Der Technikblock 2 enthält im Wesentlichen die gesamte Technik zum Spannen des Gliederarms 3 sowie eine Fixiereinrichtung 5 zum Fixieren des Gesamtsystems an einer nicht gezeigten externen Halterung. Dabei bildet der Technikblock 2 ein wiederverwendbares Technikmodul und der Gliederarm 3 zusammen mit dem Halteelement 4 ein für die Einmalverwendung konzipiertes Arbeitsmodul. Der Gliederarm 3 besteht dabei aus einer Vielzahl von halbkugelförmigen Gliederarmsegmenten 6, durch deren Mitte ein Zugseil 7 geführt ist.

In Fig. 1 befindet sich der Gliederarm 3 in einem frei beweglichen Zustand. Die einzelnen Gliederarmsegmente 6 sind somit nicht reibschlüssig gegeneinander verspannt, sondern zueinander beweglich. Dies wird insbesondere bei einer Betrachtung des Spannmechanismus innerhalb des Technikblocks 2 offensichtlich. Das Zugseil 7 ist innerhalb des Technikblocks 2 an einem Adapterabschnitt 8 befestigt, welcher wiederum an einer Kolbenmechanik 9 befestigt ist bzw. mit dieser verschraubt ist. Die Kolbenmechanik 9 ist in Fig. 1 zweiteilig ausgebildet, zusammen mit dem Adapterabschnitt 8 verschieblich innerhalb des Technikblocks 2 angeordnet und besteht aus einem distalen ersten Kolbenstangenabschnitt 10 und einem proximalen zweiten Kolbenstangenabschnitt 11, wobei der zweite Kolbenstangenabschnitt 11 an dem ersten Kolbenstangenabschnitt 10 befestigt ist bzw. mit diesem verschraubt ist.

An den proximalen Enden des ersten Kolbenstangenabschnitts 10 sowie des zweiten Kolbenstangenabschnitts 11 sind jeweils scheibenförmige Kolben 12, 13 vorgesehen. Konzentrisch zu dem ersten Kolbenstangenabschnitt 10 bzw. um diesen herum sind zwei Druckfedern 14, 15 angeordnet, die sich in proximaler Richtung hin an dem ersten Kolben 12 abstützen. In distaler Richtung hin stützen sich die Druckfedern 14, 15 an einer Trennwand 16 ab, welche an einem Grundkörperinnenteil 17 ausgebildet ist, welches wiederum ein feststehendes, unbewegliches Teil des Grundkörpers bzw. Technikblocks 2 ist.

In jeweils proximaler Richtung von den Kolben 12, 13 hin sind innerhalb des Technikblocks 2 Kolbenräume 18, 19 vorgesehen, welche in Fig. 1 mittels einer Energiequelle 26 derart pneumatisch oder hydraulisch druckbeaufschlagt sind, dass der erste Kolben 12 entgegen der Federkraft der Druckfedern 14, 15 an einen an dem Grundkörperinnenteil 17 ausgebildeten Anschlag 20 gefahren ist. In den Kolbenräumen 18, 19 wirkt somit in Fig. 1 eine ausreichend große Kraft auf die Kolben 12, 13 in distale Richtung hin, so dass die Federkraft der Druckfedern 14, 15 überwunden wird und die Kolbenmechanik 9 an den Anschlag 20 gefahren wird.

Zwischen dem ersten Kolbenraum 18 und dem zweiten Kolben 13 ist eine scheibenförmige Zwischenwand 21 eingezogen, welche zu der Kolbenmechanik 9 abgedichtet ist. Die Zwischenwand 21 ist Teil eines Führungsinnenteils 22, an welchem die Kolbenmechanik 9 bzw. insbesondere die Kolben 12, 13 geführt sind.

In Fig. 2 ist der Gliederarm 3 in einem gespannten bzw. arretierten Zustand gezeigt. Die Gliederarmsegmente 6 sind in Fig. 2 reibschlüssig gegeneinander verspannt und zwischen einem distalen Gliederarmendsegment 23, an welchem das Zugseil 7 befestigt ist, und einem feststehenden proximalen Endsegment 24 eingeklemmt.
Bei Betrachtung des Spannmechanismus in Fig. 2 wird deutlich, dass die Kolbenräume 18, 19 nun nicht mehr hydraulisch oder pneumatisch druckbeaufschlagt sind, da sich der Kolben 12 von dem Anschlag 20 wegbewegt hat und die Druckfedern 14, 15 nun für einen Verspannung der Gliederarmsegmente 6 sorgen. Die Druckfedern 14, 15 drücken somit derart auf den ersten Kolben 12, dass sich die gesamte Kolbenmechanik 9 von der feststehenden Trennwand 16 in proximale Richtung hin weggeschoben wird. Dies geschieht automatisch, sobald keine hydraulische oder pneumatische Druckbeaufschlagung auf die Kolben 12, 13 ausgehend von den Kolbenräumen 18, 19 mehr vorgenommen wird.

In Fig. 2 ist der Verfahrweg ΔK der Kolbenmechanik 9 zum Spannen des Gliederarms 3 dargestellt. Der Verfahrweg ΔK entspricht der anfänglichen Längenänderung ΔL, welche zum Spannen des Gliederarms 3 erforderlich ist. In der Längenänderung ΔL ist eine anfängliche Stauchung ΔG der Gliederarmsegmente 6 sowie eine anfängliche Dehnung ΔS des Zugseils 7 bereits mit inbegriffen. In dem gespannten Zustand der Fig. 2 bzw. in dem in diesem Zustand vorliegenden Betriebspunkt weist die Kolbenmechanik 8 eine Reserve zum Nachspannen ΔR auf. In anderen Worten fährt der Kolben 13 in dem Betriebspunkt nicht gegen die Kolbenraumbegrenzung 25, sondern es ist immer noch eine Federwegreserve ΔR vorhanden.

Kommt es nun mit zunehmender Laufzeit in dem gespannten bzw. arretierten Betrieb des Gliederarms 3 zu einer fortschreitenden Stauchung der Gliederarmsegmente 6 sowie zu einer fortschreitenden Dehnung des Zugseils 7, werden derartige Längenänderungen umgehend bzw. simultan mit jeder infinitesimal kleinen Längenänderung dadurch ausgeglichen, dass die Kolbenmechanik 9 weiterhin in proximale Richtung hin aufgrund der noch vorliegenden Federwegreserve ΔR mit Hilfe der beiden Druckfedern 14, 15 verschiebbar ist. Der gesamte mögliche Verfahrweg ΔV setzt sich somit demnach aus dem anfänglichen Verfahrweg ΔK und der zu diesem Zeitpunkt vorliegenden Federwegreserve ΔR zusammen. Folglich ist ΔV = ΔK + ΔR.

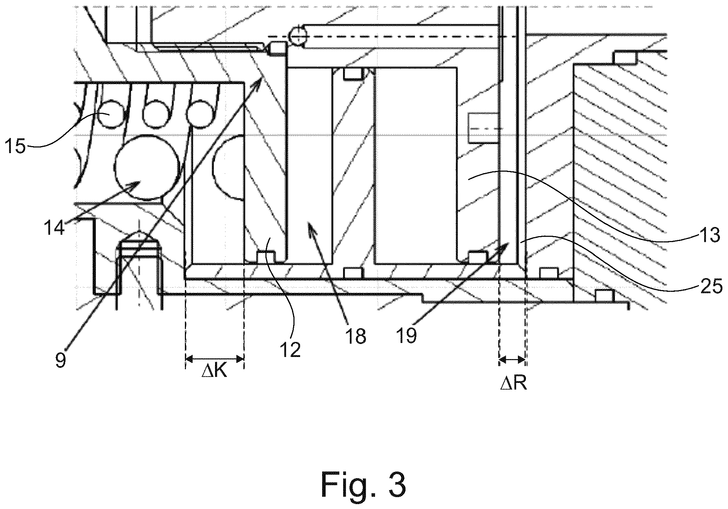

Dies ist insbesondere in Fig. 3 noch besser veranschaulicht. Hier wird noch einmal ersichtlich, dass der Kolben 12 in dem gespannten Zustand des Gliederarms 3 von dem Anschlag 20 weg in proximale Richtung um den anfänglichen Verfahrweg ΔK verschoben ist. In diesem Zustand, in welchem die Kolbenräume 18, 19 nicht hydraulisch oder pneumatisch beaufschlagt sind, liegt nach dem anfänglichen Verfahrweg ΔK weiterhin eine Reserve zum Nachspannen bzw. eine Federwegreserve ΔR vor. Über die Federwegreserve ΔR können Stauchungen der Gliederarmsegmente 6 sowie Dehnungen des Zugseils 7 ausgeglichen werden, und zwar solange bis der zweite Kolben 13 gegen die Kolbenraumbegrenzung 25 fährt.

Die Federwegreserve ΔR ist dabei insbesondere derart gewählt, dass die in dem Betrieb des Gliederarms 3 auftretenden Dehnungen und Stauchungen in jedem Fall ausgeglichen werden können. Demnach ist die Federwegreserve ΔR von der Steifigkeit bzw. Festigkeit des Zugseils 7 und der Gliederarmsegmente 6 und somit insbesondere von dem jeweilig verwendeten Material abhängig. Somit können die zu erwartenden Dehnungen bzw. Stauchungen erfindungsgemäß experimentell, beispielsweise durch Zugversuche, für beliebige Materialien bestimmt werden und die Federwegreserve ΔR dementsprechend ausgelegt werden.

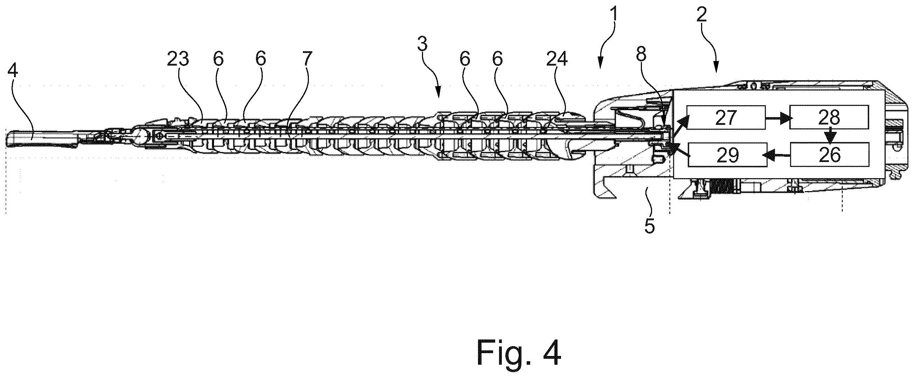

Fig. 4 zeigt eine Längsschnittansicht der erfindungsgemäßen chirurgischen Vorrichtung gemäß einer zweiten bevorzugten Ausführungsform, in welcher sowohl ein Spannen, als auch ein automatisches Nachspannen, als auch ein Lösen des Spannmechanismus durch eine Energieeinbringung/ Krafteinbringung von einer Energiequelle 26 erfolgen. In der zweiten bevorzugten Ausführungsform wird der automatische Nachspannmechanismus innerhalb des Technikblocks 2 wie folgt realisiert: Eine Erfassungseinrichtung 27, beispielsweise ein Kraftsensor, erfasst in dem arretierten Zustand des Gliederarms 3 einen Spannzustand des an dem Adapterabschnitt 8 befestigten Zugseils 7 und gibt die erfassten Daten an ein elektrisches Steuergerät 28 weiter, welches eine Änderung in dem Spannzustand überwacht. Das elektrische Steuergerät 28 steuert eine Energiequelle 26/ eine Energie-/ Krafteinbringung von der Energiequelle 26. Die Energiequelle 26 stellt einem elektrischen Stellmotor 29 Energie zur Verfügung, welcher wiederum das Zugseil betätigt/ nachspannt. Ansonsten gilt die Beschreibung zu den Fig. 1 bis 3 analog für Fig. 4.

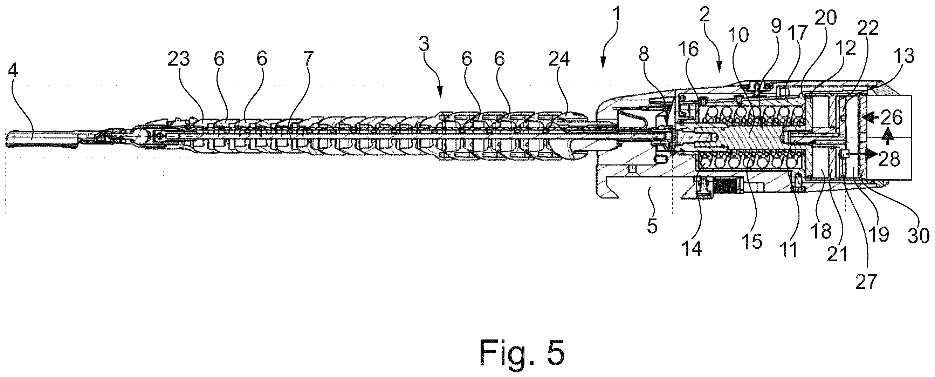

Fig. 5 zeigt eine Längsschnittansicht der erfindungsgemäßen chirurgischen Vorrichtung gemäß einer dritten bevorzugten Ausführungsform in einem beweglichen Zustand des Gliederarms 3. In Fig. 5 sind die Kolbenräume 18, 19 derart pneumatisch oder hydraulisch druckbeaufschlagt (siehe Fig. 1), dass der erste Kolben 12 entgegen der Federkraft der Druckfedern 14, 15 an einem an dem Grundkörperinnenteil 17 ausgebildeten Anschlag 20 gefahren ist. Wird in dem in Fig. 5 gezeigten Zustand die hydraulische oder pneumatische Energie weggenommen/ abgeschaltet, fährt der zweite Kolben 13 gegen einen linear beweglichen Anschlag 30. Der flexible Arm 3 befindet sich dann in einem gespannten/ arretierten Zustand. Der automatische Nachspannmechanismus wird in der dritten Ausführungsform wie folgt realisiert: Eine an dem zweiten Kolben 13 vorgesehene Erfassungseinrichtung 27, beispielsweise ein Kraftsensor, erfasst einen Spannzustand, welcher von einem elektrischen Steuergerät 28 überwacht wird. Liegt keine ausreichende Spannung mehr vor, kann der linear bewegliche Anschlag 30 mittels einer Energiequelle 26 linear in Spannrichtung verschoben werden. Bei einem Verschieben des linear beweglichen Anschlags 30 wird eine Federwegreserve ΔR freigegeben. Ansonsten gilt die Beschreibung zu den Fig. 1 bis 3 analog für die Fig. 5.

### Bezugszeichenliste

- 1: chirurgische Vorrichtung
- 2: Technikblock
- 3: Gliederarm
- 4: Halteelement
- 5: Fixiereinrichtung
- 6: Gliederarmsegmente
- 7: Zugseil
- 8: Adapterabschnitt
- 9: Kolbenmechanik
- 10: erster Kolbenstangenabschnitt
- 11: zweiter Kolbenstangenabschnitt
- 12: erster Kolben
- 13: zweiter Kolben
- 14: Druckfeder
- 15: Druckfeder
- 16: Trennwand
- 17: Grundkörperinnenteil
- 18: erster Kolbenraum
- 19: zweiter Kolbenraum
- 20: Anschlag
- 21: Zwischenwand
- 22: Führungsinnenteil
- 23: Gliederarmendsegment
- 24: Endsegment
- 25: Kolbenraumbegrenzung
- 26: Energiequelle
- 27: Erfassungseinrichtung
- 28: Steuergerät
- 29: Stellmotor
- 30: linear beweglicher Anschlag
- ΔG: anfängliche Stauchung
- ΔK: anfänglicher Verfahrweg der Kolbenmechanik
- ΔL: anfängliche Längenänderung
- ΔR: Federwegreserve/ Reserve zum Nachspannen
- ΔS: anfängliche Dehnung
- ΔV: gesamter möglicher Verfahrweg

## Patentansprüche

1. Chirurgische Vorrichtung (1) zum Stabilisieren von Gewebe oder zum Positionieren von Organen oder zum Positionieren und Halten von chirurgischen Instrumenten und Geräten während eines chirurgischen Eingriffs, mit einem Grundkörper (2) und einem, insbesondere an dem Grundkörper (2) befestigten oder befestigbaren, flexiblen Arm (3), insbesondere Gliederarm, welcher in unterschiedliche Positionen und/oder Lagen bringbar ist und welcher über einen Spannmechanismus in einer gewünschten Stellung arretierbar ist, wobei ein Spannen und/oder Lösen des Spannmechanismus selbsttätig und/oder mittels einer Energiequelle erfolgt, **dadurch gekennzeichnet, dass** in einem arretierten Zustand des flexiblen Arms (3) der Spannmechanismus eine Nachspannreserve (ΔR), insbesondere einen Nachspannreserveweg (ΔR), aufweist und der flexible Arm (3) zur Aufrechterhaltung seines arretierten Zustands, insbesondere durch eine Energieeinbringung von der Energiequelle oder selbsttätig, unter Ausnutzung der Nachspannreserve (ΔR) automatisch nachspannbar ist.

2. Chirurgische Vorrichtung (1) nach Anspruch 1, **dadurch gekennzeichnet, dass** das Spannen des Spannmechanismus selbsttätig mittels Federkraft und das Lösen des Spannmechanismus mittels der Energiequelle erfolgt, wobei die Nachspannreserve (ΔR) durch eine in dem arretierten Zustand des flexiblen Arms (3) vorliegende Federwegreserve (ΔR) ausgebildet ist.

3. Chirurgische Vorrichtung (1) nach Anspruch 1, **gekennzeichnet ferner durch** ein elektrisches Steuergerät, welches in dem arretierten Zustand des flexiblen Arms (3) eine Änderung, insbesondere eine Verminderung, in einem Spannzustand des Spannmechanismus erfasst und basierend auf dem Erfassungsergebnis, insbesondere durch eine Energieeinbringung von der Energiequelle, ein automatisches Nachspannen des Spannmechanismus steuert.

4. Chirurgische Vorrichtung (1) nach Anspruch 3, **gekennzeichnet ferner durch** einen elektrischer Stellmotor, welcher eine Erfassungseinrichtung, insbesondere einen Kraftsensor, aufweist, welche die Änderung, insbesondere die Verminderung, in dem Spannzustand des Spannmechanismus erfasst, und welcher auf der Grundlage einer Steuerung durch das elektrische Steuergerät und basierend auf dem Erfassungsergebnis der Erfassungseinrichtung den flexiblen Arm (3) automatisch nachspannt.

5. Chirurgische Vorrichtung (1) nach einem der vorhergehenden Ansprüche, **dadurch gekennzeichnet, dass** die Nachspannreserve (ΔR) durch einen in dem Grundkörper (2), insbesondere mittels der Energiequelle, linear verschieblichen Anschlag ausgebildet ist oder freigegeben wird.

6. Chirurgische Vorrichtung (1) nach einem der vorhergehenden Ansprüche, **dadurch gekennzeichnet, dass** der Spannmechanismus ein Zugseil (7), welches durch eine Vielzahl von zueinander beweglichen Gliederelementen (6) des flexiblen Arms (3) geführt ist und über welches die Gliederelemente (6) reibschlüssig gegeneinander verspannbar sind, und eine selbsttätig und/oder mittels der Energiequelle betätigbare Stellmechanik (9) zum Betätigen des Zugseils (7) aufweist.

7. Chirurgische Vorrichtung (1) nach Anspruch 6, **dadurch gekennzeichnet, dass** die Stellmechanik (9) innerhalb des Grundkörpers (2) zum Spannen und/oder Lösen des Spannmechanismus linear beweglich ist und die Nachspannreserve (ΔR) durch eine in dem arretierten Zustand des flexiblen Arms (3) weiterhin vorliegende lineare Verschieblichkeit der Stellmechanik (9) in Spannrichtung auf einem Nachspannreserveweg (ΔR) ausgebildet ist.

8. Chirurgische Vorrichtung (1) nach Anspruch 6 oder 7, **dadurch gekennzeichnet, dass** die Stellmechanik (9) nach Art einer Zylinder-Kolben-Mechanik ausgebildet ist, mit zumindest einem, vorzugsweise zwei Kolbenräumen (18, 19), in welchen eine hydraulische oder pneumatische Energie zum Spannen und/oder Lösen und/oder Nachspannen des Spannmechanismus einbringbar ist, wobei das Spannen und Nachspannen des Spannmechanismus vorzugsweise ausschließlich über zumindest eine, vorzugsweise zwei Druckfedern (14,15) oder Zugfedern erfolgt.

9. Chirurgische Vorrichtung (1) nach Anspruch 8, **dadurch gekennzeichnet, dass** das Spannen und Nachspannen des Spannmechanismus zusätzlich zu der Federkraft durch Einbringen der hydraulischen oder pneumatischen Energie, insbesondere durch eine Kraftaufbringung in Spannrichtung, erfolgt und das Lösen des Spannmechanismus ausschließlich durch Einbringen der hydraulischen oder pneumatischen Energie, insbesondere durch eine Kraftaufbringung entgegen der Spannrichtung, erfolgt.

## Claims

1. A surgical device (1) for stabilizing tissue or for positioning organs or for positioning and holding surgical instruments and devices during a surgical intervention, comprising a main body (2) and a flexible arm (3), in particular an articulated arm, which in particular is or can be fastened to the main body (2), which can be brought into different positions and/or locations and which can be locked in a desired positioning by means of a tightening mechanism, the tightening mechanism being tightened and/or released self-actingly and/or by means of an energy source, **characterized in that,** in a locked state of the flexible arm (3), the tightening mechanism has a retightening reserve (ΔR), in particular a retightening reserve travel (ΔR), and the flexible arm (3) can be automatically retightened in order to maintain its locked state, in particular by an introduction of energy from the energy source or self-actingly, by utilization of the retightening reserve (ΔR).

2. The surgical device (1) according to claim 1, **characterized in that** the tightening of the tightening mechanism is carried out self-actingly by means of spring force and the releasing of the tightening mechanism is carried out by means of the energy source, the retightening reserve (ΔR) being formed by a spring travel reserve (ΔR) available in the locked state of the flexible arm (3).

3. The surgical device (1) according to claim 1, **further characterized by** an electric control unit which, in the locked state of the flexible arm (3), detects a change, in particular a reduction, in a tightening state of the tightening mechanism and controls an automatic retightening of the tightening mechanism based on the detection result, in particular by an introduction of energy from the energy source.

4. The surgical device (1) according to claim 3, **further characterized by** an electric actuating motor which has a detection device, in particular a force sensor, which detects the change, in particular the reduction, in the tightening state of the tightening mechanism and which automatically retightens the flexible arm (3) on the basis of a control by the electric control unit and based on the detection result of the detection device.

5. The surgical device (1) according to one of the preceding claims, **characterized in that** the retightening reserve (ΔR) is formed or released by a stop which can be linearly displaced in the main body (2) in particular by means of the energy source.

6. The surgical device (1) according to one of the preceding claims, **characterized in that** the tightening mechanism comprises a traction cable (7) which is passed through a plurality of mutually movable articulated segments (6) of the flexible arm (3) and via which the articulated segments (6) can be frictionally clamped against each other, and an actuating mechanism (9) which can be actuated self-actingly and/or by means of the energy source for actuating the traction cable (7).

7. The surgical device (1) according to claim 6, **characterized in that** the actuating mechanism (9) is linearly movable within the main body (2) for tightening and/or releasing the tightening mechanism, and the retightening reserve (ΔR) is formed by a linear displaceability, which is still present in the locked state of the flexible arm (3), of the actuating mechanism (9) in the tightening direction on a retightening reserve travel (ΔR).

8. The surgical device (1) according to claim 6 or 7, **characterized in that** the actuating mechanism (9) is designed in the manner of a cylinder-piston mechanism, comprising at least one, preferably two, piston chambers (18, 19) into which hydraulic or pneumatic energy can be introduced for tightening and/or releasing and/or retightening the tightening mechanism, wherein the tightening mechanism is tightened and retightened preferably exclusively by at least one, preferably two, compression springs (14, 15) or tension springs.

9. The surgical device (1) according to claim 8, **characterized in that** the tightening and retightening of the tightening mechanism is effected in addition to the spring force by introducing the hydraulic or pneumatic energy, in particular by applying a force in the tightening direction, and the releasing of the tightening mechanism is effected exclusively by introducing the hydraulic or pneumatic energy, in particular by applying a force against the tightening direction.

## Revendications

1. Dispositif chirurgical (1) pour stabiliser un tissu tissé ou pour positionner des organes ou pour positionner et maintenir des instruments et appareils chirurgicaux pendant une intervention chirurgicale, avec un corps de base (2) et un bras flexible (3), en particulier un bras articulé fixé ou pouvant être fixé au corps de base (2), qui peut être amené dans différentes positions et/ou emplacements et peut être bloqué dans une position souhaitée au moyen d'un mécanisme de tension, la mise en tension et/ou le relâchement du mécanisme de tension s'effectuant automatiquement et/ou au moyen d'une source d'énergie, **caractérisé en ce que,** dans un état bloqué du bras flexible (3), le mécanisme de tension présente une réserve d'ajustement de tension (ΔR), en particulier une course de réserve d'ajustement de tension (ΔR), et le bras flexible (3) peut être automatiquement rétracté pour maintenir son état bloqué, en particulier par un apport d'énergie de la source d'énergie ou automatiquement en utilisant la réserve d'ajustement de tension (ΔR).

2. Dispositif chirurgical (1) selon la revendication 1, **caractérisé en ce que** la tension du mécanisme de tension s'effectue automatiquement au moyen d'une force de ressort et le relâchement du mécanisme de tension s'effectue au moyen de la source d'énergie, la réserve d'ajustement de tension (ΔR) étant formée par une réserve de course de ressort (ΔR) présente dans l'état bloqué du bras flexible (3).

3. Dispositif chirurgical (1) selon la revendication 1, **caractérisé en outre par** un dispositif de commande électrique qui, à l'état bloqué du bras flexible (3), détecte un changement, en particulier une réduction, de l'état de tension du mécanisme de tension et, sur la base du résultat de détection, en particulier par un apport d'énergie de la source d'énergie, commande un ajustement de tension automatique du mécanisme de tension.

4. Dispositif chirurgical (1) selon la revendication 3, **caractérisé en outre par** un servomoteur électrique qui présente un appareil de détection, en particulier un capteur de force, qui détecte le changement, en particulier la réduction, de l'état de tension du mécanisme de tension, et qui, sur la base d'une commande par le dispositif de commande électrique et sur la base du résultat de détection de l'appareil de détection, ajuste automatiquement la tension du bras flexible (3).

5. Dispositif chirurgical (1) selon l'une quelconque des revendications précédentes, **caractérisé en ce que** la réserve d'ajustement de tension (ΔR) est formée ou relâchée par une butée déplaçable linéairement dans le corps de base (2), en particulier au moyen de la source d'énergie.

6. Dispositif chirurgical (1) selon l'une quelconque des revendications précédentes, **caractérisé en ce que** le mécanisme de tension présente un câble de traction (7) qui est guidé par une pluralité d'éléments de liaison (6) du bras flexible (3) mobiles les uns par rapport aux autres et au moyen duquel les éléments de liaison (6) peuvent être tendus par friction les uns par rapport aux autres, et un mécanisme de réglage (9), qui peut être actionné automatiquement et/ou au moyen de la source d'énergie, pour actionner le câble de traction (7).

7. Dispositif chirurgical (1) selon la revendication 6, **caractérisé en ce que** le mécanisme de réglage (9) est mobile linéairement à l'intérieur du corps de base (2) pour tendre et/ou relâcher le mécanisme de tension, et la réserve d'ajustement de tension (ΔR) est formée par une possibilité de déplacement linéaire du mécanisme de réglage (9) dans la direction de tension sur une course de réserve d'ajustement de tension (ΔR) qui est encore présente à l'état bloqué du bras flexible (3).

8. Dispositif chirurgical (1) selon la revendication 6 ou 7, **caractérisé en ce que** le mécanisme de réglage (9) est formé à la manière d'un mécanisme à cylindre et piston, avec au moins une, de préférence deux chambres de piston (18, 19) dans lesquelles de l'énergie hydraulique ou pneumatique peut être introduite pour tendre et/ou relâcher et/ou retenir le mécanisme de tension, la tension et la retenue du mécanisme de tension s'effectuant de préférence exclusivement par l'intermédiaire d'au moins un, de préférence deux ressorts de pression (14, 15) ou ressorts de traction.

9. Dispositif chirurgical (1) selon la revendication 8, **caractérisé en ce que** la mise en tension et l'ajustement de tension du mécanisme de tension s'effectuent en plus de la force de ressort par l'introduction de l'énergie hydraulique ou pneumatique, en particulier par une application de force dans la direction de tension, et le relâchement du mécanisme de tension s'effectue exclusivement par l'introduction de l'énergie hydraulique ou pneumatique, en particulier par une application de force dans la direction opposée à la direction de tension.
